(19) Europäisches Patentamt
European Patent Office
Office européen des brevets

(11) **EP 3 797 174 B1**

(12) **EUROPEAN PATENT SPECIFICATION**

(45) Date of publication and mention
of the grant of the patent:
**03.07.2024 Bulletin 2024/27**

(21) Application number: **19725656.3**

(22) Date of filing: **09.05.2019**

(51) International Patent Classification (IPC):
***G01N 33/574*** *(2006.01)*

(52) Cooperative Patent Classification (CPC):
**C12Q 1/689; G01N 33/574; G01N 33/689;
G01N 33/6893;** C12Q 2600/158

(86) International application number:
**PCT/EP2019/061967**

(87) International publication number:
**WO 2019/224012 (28.11.2019 Gazette 2019/48)**

(54) **METHOD FOR PREDICTING THE OUTCOME OF AN ASSISTED REPRODUCTIVE TECHNOLOGY PROCEDURE**

VERFAHREN ZUR VORHERSAGE DES ERGEBNISSES EINES ASSISTIERTEN REPRODUKTIONSTECHNOLOGIEVERFAHRENS

PROCÉDÉ POUR PRÉDIRE LE RÉSULTAT D'UNE INTERVENTION DE TECHNOLOGIE DE PROCRÉATION ASSISTÉE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.05.2018 EP 18173578**

(43) Date of publication of application:
**31.03.2021 Bulletin 2021/13**

(73) Proprietor: **ARTPred B.V.**
**1438AX Oude Meer (NL)**

(72) Inventors:
• **DE JONGE, Jonathan Dennis**
**1438AX Oude Meer (NL)**
• **BUDDING, Dries**
**1438AX Oude Meer (NL)**
• **DE MÖNNINK, Joep**
**1438AX Oude Meer (NL)**

(74) Representative: **Algemeen Octrooi- en
Merkenbureau B.V.
P.O. Box 645
5600 AP Eindhoven (NL)**

(56) References cited:
**EP-A1- 1 985 712 WO-A1-2013/025095**

• **T. HAAHR ET AL: "Abnormal vaginal microbiota may be associated with poor reproductive outcomes: a prospective study in IVF patients", HUMAN REPRODUCTION, vol. 31, no. 4, 23 February 2016 (2016-02-23), pages 795-803, XP055492354, GB ISSN: 0268-1161, DOI: 10.1093/humrep/dew026**
• **J. MANGOT-BERTRAND ET AL: "Molecular diagnosis of bacterial vaginosis: impact on IVF outcome", EUROPEAN JOURNAL OF CLINICAL MICROBIOLOGY & INFECTIOUS DISEASES., vol. 32, no. 4, 7 November 2012 (2012-11-07), pages 535-541, XP055492356, DE ISSN: 0934-9723, DOI: 10.1007/s10096-012-1770-z**
• **HELMY SELMAN ET AL: "Examination of bacterial contamination at the time of embryo transfer, and its impact on the IVF/pregnancy outcome", JOURNAL OF ASSISTED REPRODUCTION AND GENETICS, KLUWER ACADEMIC PUBLISHERS-PLENUM PUBLISHERS, NE, vol. 24, no. 9, 17 July 2007 (2007-07-17), pages 395-399, XP019528306, ISSN: 1573-7330, DOI: 10.1007/S10815-007-9146-5 cited in the application**

- **ECKERT L O ET AL: "Relationship of vaginal bacteria and inflammation with conception and early pregnancy loss following in-vitro fertilization", INFECTIOUS DISEASES IN OBSTETRICS AND GYNECOLOGY, WILEY-LISS, NEW YORK, NY, US, vol. 11, no. 1, 1 January 2003 (2003-01-01), pages 11-17, XP002671987, ISSN: 1064-7449, DOI: 10.1155/S1064744903000024**
- **KOEDODDER R. ET AL.,: "The vaginal microbiome as predictor fro in vitro fertilization with or without intracytoplasmic sperm injection outocme, a prospective study", HUMAN REPRODUCTION, vol. 33, no. Suppl. 1, 1 July 2018 (2018-07-01), page i113, XP002791934,**

Remarks:
    The complete document including Reference Table(s) and the Sequence Listing(s) can be downloaded from the EPO website

**Description**

Field of the invention

The invention relates to the field of human reproduction, more in particular to situations in which human reproduction is failing. The present invention provides a reliable and highly accurate method for predicting the chance that an assisted reproductive technology (ART) procedure, such as an in vitro fertilization and intra-cytoplasmic sperm injection (ICSI) procedure will not lead to a successful pregnancy. The description also provides means and methods for predicting the chance that an assisted reproductive technology (ART) procedure, such as an in vitro fertilization and intra-cytoplasmic sperm injection (ICSI) procedure will lead to a successful pregnancy.

Background of the invention

Sub-fertility affects 10 to 15% of couples in the western world. This sub-fertility can in half of the cases be attributed to causes related to the female reproductive system, in 20-26% to the male and in 25-30% the cause is unknown (Evers, J.L., 2002, Lancet 360:151-159). Many couples turn to an assisted reproductive technology (ART) procedure such as in vitro fertilization (IVF) or intra-cytoplasmatic sperm injection (ICSI) to fulfil their child-wish.

The success rate of these techniques is around 25% per started cycle (Andersen, A. et al. 2007, Hum. Reprod. 22:1513-1525). It would be of great emotional and economical benefit if this success rate could be improved.

Moreover, in view of the personal and societal burden of ART procedures, it is desirable to identify couples with a low chance for success very early on in the procedure, so that they can be offered alternative procedures to fulfill their child wish.

Thus both for improving the treatments and for deciding in individual cases whether to proceed there is a need for models that can accurately predict if a woman will not become pregnant and give live birth after IVF/ICSI.

For over a decade, models have been available that predict the chance of live birth on the basis of clinical data including age, number of previous failed IVF attempts and probable reason for infertility (Templeton, W. et al., 1996, Lancet 348:1402-1406). Nelson and Lawlor (Nelson, S.M. and Lawlor D.A. 2011, PLOS Medicine 8:1-10) developed a model based on data from over 140.000 women, using stratification on age and cause of infertility, the procedure (to be) used, source of the egg and duration of the child wish. Selman et al., (J. Assisted Reproduction and Genetics 24: 395-399 (2007)) discloses the detection of Lactobacillus and Staphylococcus in relation to IVF/pregnancy outcome. EP 2742359 B1 describes a method for predicting the chance of a successful or unsuccessful pregnancy in a subject, based on the relative amount of bacteria belonging to the group of lactobacillaceae and bacteria belonging to a species of Staphylococcus in a urine or vaginal sample. Furthermore Haahr et al., Human Reproduction, 31, pp. 795-803, 2016; Mangot-Bertrand et al., Eur.J. Clin. Microbiol. and Infectious Diseases, 32, pp.535-54, 2012; Selman et al., J. assisted Reproduction and Genetics, 24, 395-399 (2007); Eckert et al., Inf. Disease in obstetrics and Gynecology, 11, pp.11-17, 2003; WO2013025095 A1; EP1985712 A1, describe methods for evaluating the outcome of a pregnancy and infertilisation.

Nevertheless, there remains a need for better, more reliable and easy-to-use methods for limiting the number of unnecessary ART procedures and predicting the chance of an unsuccessful outcome of an ART procedure. This would help to reduce costs in healthcare. Moreover, women with a child wish could be directed to alternative solutions earlier.

Summary of the invention

The invention is defined by the appended claims. Furthermore a method for predicting the likelihood that an assisted reproductive technology (ART) procedure will not result in a pregnancy, wherein a sample from a female mammalian subject taken before or during the ART procedure, is analyzed for the presence of Gardnerella vaginalis IST1 and additionally for at least one of the following parameters:

  a) relative abundance of Lactobacillus species
  b) relative abundance of Lactobacillus jensenii,
  c) relative abundance of Proteobacteria,

and wherein it is concluded that the subject has a high likelihood of not becoming pregnant as a result of the ART procedure if the sample comprises Gardnerella vaginalis IST1 and at least one of the following applies:

  I. the relative abundance of Lactobacillus species is below a value chosen between 15% and 25% or
  II. the relative abundance of Lactobacillus jensenii is above a value chosen between 25% and 45% or

III. the relative abundance of Proteobacteria is above a value chosen between 18% and 38%.

The invention also relates to a kit for performing a method according to the invention comprising a forward primer CTGGATCACCTCCTTTCTAWG (SEQ ID NO: 1) and a reverse primer AGGCATCCRCCATGCGCCCT (SEQ ID NO: 2) for the detection of an amplification product of Gardnerella vaginalis IST1 DNA wherein the Gardnerella vaginalis IST1 DNA amplification product has a length of 428 - 430 nucleotides, and wherein W denotes an A or a T and wherein R denotes an A or a G, is described.

We provide herein a method for predicting the likelihood that an assisted reproductive technology (ART) procedure will not result in a pregnancy, wherein a sample from a female mammalian subject taken before or during the ART procedure, is analyzed for the presence of Gardnerella vaginalis and additionally for at least one of the following parameters:

a) relative abundance of Lactobacillus species
b) relative abundance of Lactobacillus jensenii,
c) relative abundance of Proteobacteria,

and wherein it is concluded that the subject has a high likelihood of not becoming pregnant as a result of the ART procedure if the sample comprises Gardnerella vaginalis IST1 and at least one of the following applies:

I. the relative abundance of Lactobacillus species is below a value chosen between 15% and 25% or
II. the relative abundance of Lactobacillus jensenii is above a value chosen between 25% and 45% or
III. the relative abundance of Proteobacteria is above a value chosen between 18% and 38%.

The values chosen between 15% and 25%, 25% and 45% and 18% and 38% respectively are also often referred to as a cut-off values or a threshold values. It may be chosen such that the method provides the desired specificity and sensitivity. A skilled person is well aware of the meets and bounds of determining a suitable value.

The presence of Gardnerella vaginalis is determined as well as the relative abundance of Lactobacillus species, the relative abundance of Lactobacillus jensenii and the relative abundance of Proteobacteria.

In a further preferred embodiment, the invention relates to a method as described above, wherein the Gardnerella vaginalis is Gardnerella vaginalis IST1. Gardnerella vaginalis IST1 is defined herein as a specific Gardnerella species that may be identified by performing vaginal microbial population analysis using amplification of the intergenic spaces (IS), according to the protocol provided by the manufacturer (IS-pro technique, IS-Diagnostics, Amsterdam, the Netherlands). IS-pro is an eubacterial technique based on the detection and categorisation of the length of the 16S-23S rRNA gene IS region. The length of this IS region is specific for each microbial species. Gardnerella vaginalis IST1 is hereby further defined as a species of Gardnerella vaginalis that results in a specific IS-fragment with a length of 428, 429 or 430 nucleotides when primers according to SEQ ID NO: 1 and SEQ ID NO: 2 are used.

The term ART procedure is used herein to indicate an artificial reproductive technology. In particular, the term relates to in Vitro Fertilization (IVF), Intra Cytoplasmic Sperm Injection (ICSI) and Intra Uterine Insemination (IUI).

The term relative abundance is used to indicate a fraction of the total amount or number of bacteria in a sample. The fraction is either expressed as a percentage (%) or as a number between 0 and 1.

The term "high likelihood" in respect of predicting the chance of the success or failure of an ART procedure, is used herein to indicate that the predicted success or failure rate is higher than in the general population of women undergoing an ART procedure. In particular, the likelihood of not becoming pregnant is referred to as "increased" if the subject has a higher than 60% chance, such as 65% chance of not becoming pregnant as a result of the ART procedure if the criteria for a negative prediction as described herein are fulfilled. Higher than 65% in this respect includes for instance higher than 77%, such as 88% or higher or even 94% or higher.

Also, the likelihood of becoming pregnant is increased if the subject has a higher than 35% chance of becoming pregnant as a result of the ART procedure if the criteria for a positive prediction as described herein are fulfilled. Higher than 35% in this respect means 41%, 49% or even 50% or more.

Gardnerella is a genus of Gram-variable-staining facultative anaerobic bacteria of which Gardnerella vaginalis is the only species. The organisms are small (1.0-1.5 $\mu$m in diameter) nonspore-forming, nonmotile coccobacilli. Once classified as Haemophilus vaginalis and afterwards as Corynebacterium vaginalis, G. vaginalis grows as small, circular, convex, gray colonies on chocolate agar; it also grows on HBT agar. A selective medium for G. vaginalis is colistin-oxolinic acid blood agar. Determining the presence of Gardnerella vaginalis is preferably done by PCR, such as quantitative PCR.

Lactobacillus is a genus of Gram-positive, facultative anaerobic or microaerophilic, rod-shaped, non-spore-forming bacteria. They are a major part of the lactic acid bacteria group (i.e. they convert sugars to lactic acid). In humans, they constitute a significant component of the microbiota at a number of body sites, such as the digestive system, urinary system, and genital system. In women of European ancestry, Lactobacillus species are normally a major part of the vaginal microbiota. Lactobacillus forms biofilms in the vaginal and gut microbiota, allowing them to persist during harsh environmental conditions and maintain ample populations. Lactobacillus exhibits a mutualistic relationship with the human body as it protects the host against potential invasions by pathogens, and in turn, the host provides a source of nutrients.

The term Lactobacillus species is used herein to refer to all Lactobacillus species collectively.

Lactobacillus jensenii is a common inhabitant of the lower reproductive tract in healthy women. In a normal population, L. jensenii makes up to about 23% of vaginal microflora that is naturally occurring.

The Proteobacteria are a major phylum of bacteria. They are gram-negative bacteria. This means they do not retain the violet dye in the Gram staining protocol. In a Gram stain test, a counterstain (commonly safranin) is added after the crystal violet, colouring all gram-negative bacteria with a pink colour. The test itself is useful in classifying two distinct types of bacteria based on the structural differences of their cell walls.

Proteobacteria include a wide variety of pathogens, such as Escherichia coli, Salmonella, Vibrio, Helicobacter, and many other notable genera. Others are free-living, and include many of the bacteria responsible for nitrogen fixation. The group is defined primarily in terms of ribosomal RNA (rRNA) sequences.

There are numerous ways for determining these microorganisms and the skilled person is well aware of techniques on how to determine and quantify the relative amounts of Gardnerella, Lactobacillus species, L. jensenii and Proteobacteria in a sample. We provide herein the results of a study wherein we determined the presence and relative amounts of these bacteria in samples obtained from a population of 192 women undergoing an ART procedure.

We correlated the presence and relative amounts of these microorganisms and found that their presence and/or abundance was indicative of the success rate or failure rate of an ART procedure.

Out of 192 women, 125 did not become pregnant after the first attempt whereas 67 did become pregnant. This is a failure rate of the ART procedure of 65% and a success rate of 35% (Table 1 and Table 2).

In a method according to the invention, the relative abundance of a particular species or genus of bacteria has to be compared with a predetermined reference value or cut-off value. The predetermined reference value may be any suitable cut-off value. This process of determining a suitable cut-off value is well within the skills of a skilled person and can easily be determined empirically by the skilled person.

Preferably it is a value derived from the bacterial composition of samples obtained from a comparable population as the test population. Even more preferred is a reference value obtained from an average value of several independent experiments of ART procedures in a reference population. The skilled person is aware of the particulars of determining reference values for measuring and determining the relative abundance of bacteria.

Hence, the predetermined reference value may be empirically determined or arbitrarily chosen in order to achieve appropriate specificity and/or sensitivity of the method. A skilled person is fully aware how to choose an appropriate reference value. A skilled person will know how to alter the predetermined reference value in order to obtain the desired specificity and sensitivity of the method.

As an example, in the method described above, the first predetermined reference value may be between 15 and 25%, such as 20%, the second predetermined reference value may be between 25 and 45%, such as 35% and the third predetermined reference value may be between 18 and 38%, such as 28%.

When the first, second and third reference values were chosen as 20%, 35% and 28% respectively and the data from table 1 were combined with the data of the microbial composition of the vaginal bacterial population, it appeared that 32 of the 125 unsuccessful ART outcomes could be correctly predicted, i.e. 26% of the cases where the ART procedure failed, could be correctly predicted (Table 2).

Hence, a method according to the invention as disclosed above, produces highly reliable results, i.e. it failed to predict an unsuccessful outcome of the ART procedure in only 2 cases. In these two cases, the ART procedure resulted in a pregnancy which is considered the desired outcome; i.e. a successful outcome. The precision of the method to predict that a subject will not become pregnant, as described above, is therefore 32/34 = 94% (Table 5).

Table 2: Correlation matrix based on at least one out of 4 parameters

| Actual result of ART procedure | Prediction according to a method of the invention | | |
|---|---|---|---|
| | Not Pregnant | Pregnant | Total |
| Not Pregnant | 32 | 93 | 125 |
| Pregnant | 2 | 65 | 67 |
| Total | 34 | 158 | 192 |

If the method according to the invention as described above would have been used as an exclusion criterion in this study, then 34 women would have been excluded from this study and 158 instead of 192 women would have been allowed into the procedure, of which 65 would have become pregnant. This means that the success rate of the ART procedure in that case would have been increased from 67/192 ≈ 35% to 65/158= 41%. This is an increase of the relative efficiency of the ART procedure with 6%. An additional advantage would be that the 34 women would not have to undergo an ART procedure or procedures before they would have been offered alternative approaches.

If applied to the present population in the study as described herein, the selection procedure would have led to a reduction of the number of ART procedures with 34/192 ≈ 18%. The total average costs for an ART procedure such as IVF or ICSI are in the order of €5.000. In total, applying the method according to the invention would have saved on average €900 per IVF/ICSI patient, or in other terms, the costs of the procedure would have been reduced with 18%.

We also determined the predictive value of a method based on the presence of Gardnerella vaginalis, such as G. vaginalis IST, in particular G. vaginalis IST1, alone. It appeared that women with Gardnerella vaginalis IST1 had an 88% chance of not becoming pregnant (Table 3, Table 5). Hence, a method as described above, wherein Gardnerella vaginalis is G. vaginalis IST1 yielded good results.

Table 3: Correlation matrix based on Gardnerella vaginalis IST1

| Actual result of ART procedure | Prediction according to a method of the invention | | |
|---|---|---|---|
| | Not Pregnant | Pregnant | Total |
| Not Pregnant | 15 | 110 | 125 |
| Pregnant | 2 | 65 | 67 |
| Total | 17 | 175 | 192 |

Hence, the invention also relates to a method as described above wherein the presence of Gardnerella vaginalis, preferably G. vaginalis IST, such as G. vaginalis IST1 is determined in the sample and wherein the subject has a high likelihood of not becoming pregnant as a result of the ART procedure if the sample comprises Gardnerella vaginalis, preferably G. vaginalis IST1.

The accuracy and other features of this method as described above could even be further improved by applying a method wherein at least one of the following parameters is measured:

a) relative abundance of Lactobacillus species
b) relative abundance of Lactobacillus jensenii,
c) relative abundance of Proteobacteria,

and wherein it is concluded that the subject has a high likelihood of not becoming pregnant as a result of the ART procedure if the sample comprises Gardnerella vaginalis IST1 and at least one of the following applies:

I. the relative abundance of Lactobacillus species is below a value chosen between 15% and 25% or
II. the relative abundance of Lactobacillus jensenii is above a value chosen between 25% and 45% or
III. the relative abundance of Proteobacteria is above a value chosen between 18% and 38%.

When these criteria are applied, more samples could be correctly predicted.

As can be deducted from the data in Table 1, 34 of the total 192 samples were predicted to not succeed in becoming pregnant. Thirtytwo of these 34 samples could be correctly predicted by a method according to the invention (Table 1, Table 2). From the 32 correctly predicted samples, 15 were attributable to the presence of G. vaginalis IST1 (criterion 1). If this criterion was combined with the criterion of the relative abundance of Lactobacillus species (criterion 2) being less than 15%, then an extra 7 samples of these 32 could be correctly predicted. If criterion 1 was combined with the criterion of the relative abundance of Lactobacillus jensenii (criterion 3) being above 25%, then an extra 6 samples of these 32 could be correctly predicted. If criterion 1 was combined with the criterion of the relative abundance of Proteobacteria (criterion 4) being above 18%, then an extra 5 samples of these 32 could be correctly predicted. Other combinations of these criteria also yielded an improvement in the method. The below Table 7 provides the numbers of correct predictions depending on the criteria used. If all 4 criteria were used, all 32 samples were detected.

Table 7: Correct prediction of not becoming pregnant based on different criteria.

| Criterion | Correct prediction of not pregnant |
|---|---|
| 1 | 15/32 |

(continued)

| Criterion | Correct prediction of not pregnant |
|---|---|
| 1+2 | 22/32 |
| 1+3 | 21/32 |
| 1+4 | 20/32 |
| 1+2+3 | 28/32 |
| 1+3+4 | 26/32 |
| 1+2+3+4 | 32/32 |

We also determined the presence of two Lactobacillus species, L. crispatus and L. iners in the samples provided, using the ISPRO technique (Example 4). The results are shown in Table 4.

Lactobacillus crispatus is a common inhabitant of the lower reproductive tract in healthy women. In a normal population, L. crispatus is the dominant species in more than 30% of all women of reproductive age.

Lactobacillus iners is also a species in the genus Lactobacillus. It is a Gram-positive, catalase-negative, facultatively anaerobic rod-shaped bacterium. Lactobacillus iners is a normal inhabitant of the lower reproductive tract in healthy women. The genomes of at least 15 strains have been sequenced and encode between 1,152 and 1,506 proteins. Therewith this species has one of the smallest Lactobacillus genomes compared to other species, such as L. crispatus, which typically encodes more than twice as many proteins.

There are numerous ways for determining these microorganisms and the skilled person is well aware of techniques on how to determine and quantify the relative amounts of Lactobacillus species, L. crispatus and L. iners in a sample.

We found that these two species (L. crispatus and L. iners) were also indicative of the failure or success of an ART procedure.

In particular, it was found that a subject had a high likelihood of not becoming pregnant as a result of an ART procedure, if the relative abundance of Lactobacillus crispatus was above a fourth predetermined reference value. This fourth predetermined reference value was preferably chosen between 50 and 70%, such as 60%. When 60% was taken as the fourth reference value, 65 women fulfilled this criterion, of which 15 became pregnant as a result of the ART procedure (Table 6). This is a failure rate of 77%, which is higher than the failure rate in the entire group (Table 5).

it was also found that a subject had a high likelihood of becoming pregnant as a result of the ART procedure, if the relative abundance of Lactobacillus crispatus was below a fifth predetermined reference value. This fifth predetermined reference value was preferably chosen between 50 and 70%, such as 60%. When 60% was taken as the fifth reference value, 127 women fulfilled this criterion, of which 52 became pregnant as a result of the ART procedure (Table 6). This is a success rate of 41%, which is higher than the success rate in the entire group (Table 5). These results are shown in Table 6 and Table 5.

Table 6: Correlation matrix based on L. crispatus

| Actual result of ART procedure | Prediction according to a method of the invention | | |
|---|---|---|---|
| | Not Pregnant | Pregnant | Total |
| **Not Pregnant** | 50 | 75 | 125 |
| **Pregnant** | 15 | 52 | 67 |
| **Total** | 65 | 127 | 192 |

We also determined the relative abundance of Lactobacillus iners and found that the subject had a high likelihood of becoming pregnant as a result of the ART procedure, if the relative abundance of Lactobacillus iners was above a sixth predetermined reference value. This sixth predetermined reference value was preferably chosen between 50 and 70%, such as 60%. When 60% was taken as the sixth reference value, 38 women fulfilled this criterion, of which 19 became pregnant as a result of the ART procedure (Table 5). This is a success rate of 50%, which is higher than the success rate in the entire group (Table 5).

Hence, we describe a method for predicting the likelihood that an assisted reproductive technology (ART) procedure will not result in a pregnancy, wherein a sample from a female mammalian subject taken before or during the ART procedure, is analyzed for the relative abundance of Lactobacillus crispatus and wherein the subject has high likelihood of not becoming pregnant as a result of the ART procedure, if the relative abundance of Lactobacillus crispatus is above a fourth predetermined reference value.

We also describe a method for predicting the likelihood that an assisted reproductive technology (ART) procedure will result in a pregnancy, wherein a sample from a female mammalian subject taken before or during the ART procedure, is analyzed for the relative abundance of Lactobacillus crispatus and wherein the subject has high likelihood of becoming pregnant as a result of the ART procedure, if the relative abundance of Lactobacillus crispatus is below a fifth predetermined reference value .

We also describe a method for predicting the likelihood that an assisted reproductive technology (ART) procedure will result in a pregnancy, wherein a sample from a female mammalian subject taken before or during the ART procedure, is analyzed for the relative abundance of Lactobacillus iners and wherein the subject has high likelihood of becoming pregnant as a result of the ART procedure, if the relative abundance of Lactobacillus iners is below a sixth predetermined reference value .

We also describe herein a method for predicting the likelihood that an assisted reproductive technology (ART) procedure will result in a pregnancy, wherein a sample from a female mammalian subject taken before or during the ART procedure, is analyzed for the presence of Lactobacillus crispatus and of Lactobacillus iners, and wherein the relative amounts of L. crispatus [LC] and L. iners [LI] are determined, and wherein the likelihood of a pregnancy is increased if [LC] is below a seventh predetermined reference value and wherein

a.

$$[LC\} < (a * [LI]) + b$$

and

b.

$$[LC\} > (c * [LI]) + d$$

and

wherein a is a value between -0.55 and -0.70, b is a value between 0.80 and 0.90, c is a value between -0.50 and -0.65 and wherein d is a value between 0.3 and 0.45.

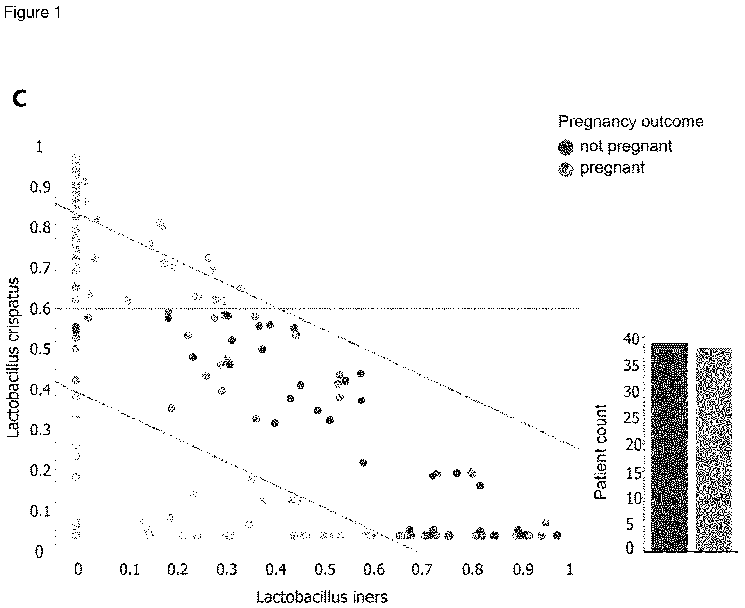

In a preferred embodiment, a = -0.62, b = 0.85, c = -0.58 and d = 0.38. In this case, 77 of the 192 women from the study described herein were found to fulfill the criterion, of which 38 (49%) became pregnant as a result of the ART procedure (Table 5). These results are graphically represented in figure 1.

Particularly good results were obtained when the fourth, fifth, sixth and seventh predetermined reference values were independently from each other chosen between 50% and 70%, even more in particular 60%.

Legend to the figure

Figure 1: Scatter plot of data obtained with one of the methods exemplified herein wherein the likelihood of a pregnancy is increased if [LC] is below 60% and wherein

$$[LC\} < (a * [LI]) + b$$

and wherein

$$[LC\} > (c * [LI]) + d$$

and wherein

a = -0.62,
b = 0.85,
c = -0.58 and

d = 0.38.

Table 5

| Criterion | # individuals fulfilling the criterion | # individuals that became pregnant as a result of ART in the population fulfilling the criterion | % success of pregnancy within the group fulfilling the criterion | % failure of pregnancy within the group fulfilling the criterion |
|---|---|---|---|---|
| Total population | 192 | 67 | 35% | 65% |
| One out of 4 parameters:<br>1) presence of Gardnerella vaginalis IST1,<br>2) abundance of Lactobacillus species <20%<br>3) abundance of L jensenii > 35%<br>4) Proteobacterium > 28% | 34 | 2 | 6% | 94% |
| L. crispatus > 60% | 65 | 15 | 23% | 77% |
| L. crispatus <60% | 127 | 52 | 41% | 59% |
| L. iners >60% | 38 | 19 | 50% | 50% |
| a.	[LC] < 0.6 AND<br>b.	[LC} < (-0.62 * [LI]) + 0.85 AND<br>c.	[LC} > (-0.58 * [LI]) + 0.38. | 77 | 38 | 49% | 51% |
| Presence of Gardnerella vaginalis IST1 | 17 | 2 | 12% | 88% |

Examples

Example 1: Study population

This prospective study of the vaginal microbiome of sub-fertile women of reproductive age was carried out in eight IVF centres in the Netherlands. The participating centres were: Erasmus Medical Centre (Rotterdam), Radboud UMC (Nijmegen), UMC Utrecht (Utrecht), VU MC (Amsterdam), Isala kliniek (Zwolle), Sint Elisabeth Ziekenhuis (Tilburg), MC Kinderwens (Leiderdorp), MUMC+ (Maastricht). Inclusions took place over an almost one year period (1st June 2015 to 31st March 2016). The protocol was approved by the Institutional Review Board of the Erasmus University Medical Centre. Written informed consent was obtained from all participants.

Women who visited the outpatient clinic of reproductive health clinic and who were expected to undergo their first IVF (with or without ICSI) procedure within two months were approached to participate in this study. Criteria to be fulfilled were: women between the ages of 20 and 44 years and having a male partner. Those excluded from the study were: women with an indication for emergency IVF because of cancer or other reasons, endometriosis AFS III/IV and pretreated with a Gonadotrophin-releasing hormone (GnRH) analogue, use of hormonal contraceptives 3 months prior to start IVF or IVF-ICSI (exclusive 3 weeks use of oral contraceptive pill for the purpose of cycle regulation) and who had a previous pregnancy or miscarriage in medical history.

In this study, 301 women were enrolled at first. Twenty-one patients were excluded on the basis of the exclusion criteria, another 86 left the study for personal or unknown reasons. Two samples were lost due to handling errors, hence the study was eventually conducted with samples from 192 individuals.

Example 2: Materials

Participants obtained a vaginal swab by themselves prior to the start of the IVF or IVF-ICSI procedure. A self-collecting method was chosen, because it is minimal invasive for the patient and therefore suitable for use in the daily practice. The vaginal samples were taken with FLOQSwabs™ (Copan Italia S.p.A., Italy) and the participants were instructed to insert the swab 3-5 centimetre into the vagina, then to rub the swab along the vaginal wall for 10-15 seconds. After this procedure the swabs were immediately placed in Eppendorf tubes filled with reduced transport fluid (RTF) buffer, obtained from IS-Diagnostics (IS-Diagnostics, Amsterdam, the Netherlands). Up to the analysis, the samples were stored at -20 to -80 °C degrees in the freezer.

Urine samples were collected in a sterile urine collecting device of 100 ml. A 10 ml sample was centrifuged for 10 minutes at 1500 RCF. The supernatant was decanted and the pellet was re-suspended in 3 ml urine. The re-suspended sample was stored for further processing at -20 degrees Celsius.

Samples were transported on dry ice from the 8 clinics to the microbiological laboratory of IS-Diagnostics, where the analyses were performed.

Example 3: DNA isolation

DNA extraction was performed from the vaginal swabs with the Chemagen (Chemagen, Baesweiler, Germany) automated DNA extraction machine using the buccal swab extraction kit according to the manufacturer's instructions. First the swabs were thawed and vortexed. 200 $\mu$l of sample was incubated with 200 $\mu$l Chemagen lysisbuffer and 10 $\mu$l Proteinase K (Qiagen, Hilden, Germany) at 56 degrees Celsius while shaking at 500 rpm. DNA was extracted using the protocol buccal Swab Prefilling. Elution of DNA was in 100$\mu$l of Chemagen Elution buffer.

DNA was extracted from concentrated urine suspensions with the Chemagen (Perkin-Elmer, Baesweiler, Germany) automated DNA extraction machine using the buccal swab extraction kit according to the manufacturer's instructions. In short, urine samples were thawed and vortexed. 200 $\mu$l of sample was incubated with 200 $\mu$l Chemagen lysis buffer and 10 $\mu$l Proteinase K at 56 degrees Celsius while shaking at 500 rpm. Elution of DNA was in 100$\mu$l of Chemagen Elution buffer.

Example 4: Interspace (IS) profiling

Amplification of the intergenic spaces (IS) regions was performed with the IS-pro assay, according to the protocol provided by the manufacturer (IS-Diagnostics, Amsterdam, the Netherlands). IS-pro is an eubacterial technique based on the detection and categorisation of the length of the 16S-23S rRNA gene IS region. The length of this IS region is specific for each microbial species. Phylum-specific fluorescently labelled PCR primers are used for taxonomic classification.

Briefly, the procedure consists of two separate standard PCRs: the first PCR mixture contains two different fluorescently labelled forward primers targeting different bacterial groups and three reverse primers providing universal coverage for those groups. The first forward primer is specific for the phyla Firmicutes, Actinobacteria, Fusobacteria, and Verrucomicrobia (FAFV), and the second labeled forward primer is specific for the phylum Bacteroidetes. A separate PCR with a labeled forward primer combined with seven reverse primers is specific for the phylum Proteobacteria [Budding, E. et al., J. Clin. Microbiol. (2016) 54: 934-943].

GeneAmp 9700 PCR system (Applied Biosystems, Foster City, CA) performed the amplications. After PCR, 5 $\mu$l of PCR product was mixed with 20 $\mu$l of formamide and 0.2 $\mu$l of custom size marker (IS-Diagnostics). DNA fragment analysis was performed on an ABI Prism 3500 genetic analyzer (Applied Biosystems). Data were analyzed with the IS-pro proprietary software suite (IS-Diagnostics), and the results are presented as microbial profiles. Automated species calling of IS-pro peaks was done with the dedicated IS-pro software suite (IS-Diagnostics), in which peaks are linked to a database containing IS-profile information of >500 microbial species. Peaks of <128 relative fluorescence units (RFU) were regarded as background noise and were discarded from further analysis. The whole procedure, from DNA isolation to analyzed data, was performed within 5 hours.

Example 5; Outcome measurement

Pregnancy outcome after the first embryo transfer (ET), was used as endpoint. Ongoing pregnancy was defined as a fetus with heart activity established with the use of an ultrasound between 7-9 weeks of gestation.

Example 6: Determination of Gardnerella vaginalis IST1

Gardnerella vaginalis IST1 was identified by performing vaginal microbial population analysis with the IS-pro technique. G.vaginalis IST1 was detected by presence of a specific IS-fragment with a length of 428-430 nucleotides.

Example 7: Determination of microbiome

Microbiome analysis was performed with the IS-pro technique as described previously [Automated Broad-Range Molecular Detection of Bacteria in Clinical Samples. Budding AE, Hoogewerf M, Vandenbroucke-Grauls CM, Savelkoul PH.. J Clin Microbiol. 2016 Apr;54(4):934-43. doi: 10.1128/JCM.02886-15. Epub 2016 Jan 13; IS-pro: high-throughput molecular fingerprinting of the intestinal microbiota., Budding AE, Grasman ME, Lin F, Bogaards JA, Soeltan-Kaersenhout DJ, Vandenbroucke-Grauls CM, van Bodegraven AA, Savelkoul PH.]

Table 1: Microbial composition of vaginal flora; prediction of chance of failure of ART procedure.

| Sample ID | G. vaginalis | Total Lactobacillus | L. jensenii | Total Proteobacterium | Prediction | Outcome | Treatment |
|---|---|---|---|---|---|---|---|
| F34 | 34.93% | 0.00% | 0.00% | 0.00% | not pregnant | not pregnant | IVF |
| B83 | 17.61% | 44.59% | 0.00% | 0.00% | not pregnant | not pregnant | ICSI |
| C1 | 4.03% | 80.36% | 80.36% | 0.00% | not pregnant | not pregnant | ICSI |
| H9 | 1.63% | 95.51% | 0.00% | 0.00% | not pregnant | not pregnant | IVF |
| B59 | 0.00% | 15.42% | 0.00% | 0.00% | not pregnant | not pregnant | ICSI |
| B17 | 5.05% | 70.82% | 4.38% | 0.00% | not pregnant | not pregnant | ICSI |
| H3 | 0.71% | 86.97% | 31.80% | 0.00% | not pregnant | not pregnant | IVF |
| B13 | 0.00% | 94.44% | 87.81% | 0.89% | not pregnant | not pregnant | ICSI |
| B15 | 0.00% | 0.42% | 0.00% | 4.72% | not pregnant | not pregnant | IVF |
| F40 | 0.00% | 0.00% | 0.00% | 5.33% | not pregnant | not pregnant | IVF |
| A18 | 0.52% | 1.38% | 0.00% | 5.42% | not pregnant | not pregnant | IVF |
| C25 | 0.00% | 0.00% | 0.00% | 8.03% | not pregnant | not pregnant | ICSI |
| B11 | 0.00% | 63.86% | 63.86% | 8.99% | not pregnant | not pregnant | ICSI |
| F23 | 0.00% | 17.49% | 0.00% | 9.85% | not pregnant | not pregnant | ICSI |
| B88 | 6.49% | 0.00% | 0.00% | 21.88% | not pregnant | not pregnant | ICSI |
| F6 | 14.48% | 37.47% | 0.00% | 29.16% | not pregnant | not pregnant | IVF |
| F16 | 0.00% | 62.27% | 0.00% | 36.91% | not pregnant | not pregnant | ICSI |
| H7 | 3.08% | 17.47% | 0.00% | 23.15% | not pregnant | pregnant | IVF |
| A32 | 0.00% | 15.93% | 0.00% | 29.43% | not pregnant | not pregnant | ICSI |
| B18 | 0.00% | 14.88% | 0.00% | 7.03% | not pregnant | not pregnant | ICSI |
| B65 | 3.05% | 23.59% | 0.00% | 22.67% | not pregnant | not pregnant | ICSI |
| D3 | 0.54% | 34.58% | 0.00% | 1.30% | not pregnant | pregnant | ICSI |
| C11 | 10.62% | 24.40% | 0.00% | 5.21% | not pregnant | not pregnant | ICSI |
| B54 | 0.51% | 31.39% | 0.00% | 6.92% | not pregnant | not pregnant | ICSI |
| B45 | 0.00% | 37.63% | 0.00% | 29.62% | not pregnant | not pregnant | IVF |

| Sample ID | G. vaginalis | Total Lactobacillus | L. jensenii | Total Proteobacterium | Prediction | Outcome | Treatment |
|-----------|--------------|---------------------|-------------|----------------------|------------|---------|-----------|
| A41 | 0.00% | 52.79% | 0.00% | 46.04% | not pregnant | not pregnant | ICSI |
| D2 | 0.00% | 98.29% | 54.36% | 0.00% | not pregnant | not pregnant | ICSI |
| C47 | 1.60% | 45.01% | 0.00% | 21.76% | not pregnant | not pregnant | ICSI |
| C10 | 0.00% | 99.61% | 49.94% | 0.00% | not pregnant | not pregnant | ICSI |
| F28 | 0.00% | 93.98% | 40.79% | 6.02% | not pregnant | not pregnant | ICSI |
| F32 | 3.12% | 76.89% | 0.00% | 2.54% | not pregnant | not pregnant | ICSI |
| F10 | 0.00% | 59.16% | 0.00% | 40.36% | not pregnant | not pregnant | IVF |
| E38 | 0.00% | 95.46% | 35.98% | 0.00% | not pregnant | not pregnant | ICSI |
| B41 | 6.45% | 68.57% | 0.00% | 0.00% | not pregnant | not pregnant | IVF |
| C4 | 0.00% | 80.13% | 0.00% | 0.00% | pregnant | not pregnant | IVF |
| G3 | 0.00% | 99.36% | 0.00% | 0.00% | pregnant | not pregnant | IVF |
| F33 | 0.00% | 76.12% | 1.08% | 0.00% | pregnant | not pregnant | ICSI |
| F42 | 0.00% | 77.08% | 12.17% | 0.00% | pregnant | not pregnant | IVF |
| E7 | 0.00% | 72.39% | 27.61% | 0.00% | pregnant | not pregnant | IVF |
| C3 | 0.00% | 98.29% | 0.00% | 0.00% | pregnant | not pregnant | IVF |
| B51 | 0.00% | 78.65% | 0.00% | 0.00% | pregnant | not pregnant | ICSI |
| E40 | 0.00% | 100.00% | 19.29% | 0.00% | pregnant | not pregnant | ICSI |
| E44 | 0.00% | 100.00% | 12.73% | 0.00% | pregnant | not pregnant | IVF |
| E47 | 0.00% | 97.84% | 0.00% | 0.00% | pregnant | not pregnant | IVF |
| B29 | 0.00% | 100.00% | 1.92% | 0.00% | pregnant | not pregnant | ICSI |
| B28 | 0.00% | 100.00% | 1.79% | 0.00% | pregnant | not pregnant | ICSI |
| B26 | 0.00% | 100.00% | 0.00% | 0.00% | pregnant | not pregnant | ICSI |
| D6 | 0.00% | 100.00% | 0.00% | 0.00% | pregnant | not pregnant | IVF |
| H5 | 0.00% | 95.46% | 3.11% | 0.94% | pregnant | not pregnant | ICSI |
| C20 | 0.00% | 58.17% | 4.18% | 1.19% | pregnant | not pregnant | ICSI |

(continued)

| Sample ID | G. vaginalis | Total Lactobacillus | L. jensenii | Total Proteobacterium | Prediction | Outcome | Treatment |
|---|---|---|---|---|---|---|---|
| C32 | 0.00% | 93.91% | 0.00% | 3.03% | pregnant | not pregnant | IVF |
| F12 | 0.00% | 89.56% | 0.00% | 3.33% | pregnant | not pregnant | ICSI |
| B67 | 0.00% | 93.28% | 0.00% | 3.65% | pregnant | not pregnant | ICSI |
| B71 | 0.00% | 95.30% | 3.84% | 4.70% | pregnant | not pregnant | IVF |
| B63 | 0.00% | 95.09% | 0.00% | 4.91% | pregnant | not pregnant | ICSI |
| B90 | 0.00% | 94.80% | 0.00% | 5.20% | pregnant | not pregnant | ICSI |
| B49 | 0.00% | 94.75% | 0.00% | 5.25% | pregnant | not pregnant | ICSI |
| E57 | 0.00% | 91.98% | 0.00% | 5.81% | pregnant | not pregnant | ICSI |
| E8 | 0.00% | 93.62% | 0.00% | 6.38% | pregnant | not pregnant | IVF |
| F38 | 0.00% | 80.94% | 0.00% | 6.60% | pregnant | not pregnant | ICSI |
| F15 | 0.00% | 92.32% | 0.00% | 7.04% | pregnant | not pregnant | IVF |
| A15 | 0.00% | 91.16% | 0.00% | 8.84% | pregnant | not pregnant | IVF |
| A22 | 0.00% | 76.68% | 0.00% | 9.37% | pregnant | not pregnant | ICSI |
| C53 | 0.00% | 90.59% | 9.00% | 9.41% | pregnant | not pregnant | ICSI |
| A21 | 0.00% | 65.89% | 0.00% | 9.53% | pregnant | not pregnant | ICSI |
| F13 | 0.00% | 90.41% | 0.00% | 9.59% | pregnant | not pregnant | IVF |
| A19 | 0.00% | 89.78% | 1.05% | 10.22% | pregnant | not pregnant | ICSI |
| A16 | 0.00% | 77.71% | 0.00% | 12.12% | pregnant | not pregnant | ICSI |
| C39 | 0.00% | 85.32% | 0.00% | 12.44% | pregnant | not pregnant | ICSI |
| F9 | 0.00% | 29.92% | 0.00% | 12.70% | pregnant | not pregnant | IVF |
| D21 | 0.00% | 85.39% | 15.74% | 14.26% | pregnant | not pregnant | ICSI |
| F17 | 0.00% | 85.66% | 1.69% | 14.34% | pregnant | not pregnant | IVF |
| A20 | 0.00% | 81.20% | 0.00% | 15.55% | pregnant | not pregnant | ICSI |
| A36 | 0.00% | 82.86% | 7.96% | 17.14% | pregnant | not pregnant | ICSI |
| D23 | 0.00% | 79.02% | 3.82% | 20.15% | pregnant | not pregnant | ICSI |

| Sample ID | G. vaginalis | Total Lactobacillus | L. jensenii | Total Proteobacterium | Prediction | Outcome | Treatment |
|---|---|---|---|---|---|---|---|
| F36 | 0.00% | 99.44% | 0.00% | 0.00% | pregnant | pregnant | ICSI |
| B76 | 0.00% | 100.00% | 0.00% | 0.00% | pregnant | pregnant | IVF |
| B30 | 0.00% | 65.88% | 0.00% | 0.00% | pregnant | pregnant | ICSI |
| B40 | 0.00% | 28.77% | 24.53% | 0.00% | pregnant | pregnant | ICSI |
| B37 | 0.00% | 77.53% | 0.00% | 0.00% | pregnant | pregnant | ICSI |
| B6 | 0.00% | 96.10% | 0.00% | 0.00% | pregnant | pregnant | ICSI |
| B72 | 0.00% | 87.70% | 32.73% | 0.79% | pregnant | pregnant | ICSI |
| B64 | 0.00% | 99.21% | 0.00% | 0.79% | pregnant | pregnant | ICSI |
| B84 | 0.00% | 70.61% | 14.28% | 0.80% | pregnant | pregnant | ICSI |
| B3 | 0.00% | 99.19% | 28.33% | 0.81% | pregnant | pregnant | IVF |
| C14 | 0.00% | 75.92% | 16.39% | 1.12% | pregnant | pregnant | ICSI |
| F37 | 0.00% | 85.77% | 0.00% | 1.43% | pregnant | pregnant | ICSI |
| C36 | 0.00% | 92.31% | 1.14% | 2.42% | pregnant | pregnant | ICSI |
| C33 | 0.00% | 97.29% | 0.00% | 2.71% | pregnant | pregnant | IVF |
| B44 | 0.00% | 91.38% | 0.00% | 2.87% | pregnant | pregnant | ICSI |
| B12 | 0.00% | 96.88% | 0.00% | 3.12% | pregnant | pregnant | IVF |
| A3 | 0.00% | 92.46% | 0.00% | 5.65% | pregnant | pregnant | ICSI |
| A5 | 0.00% | 67.98% | 23.08% | 7.01% | pregnant | pregnant | ICSI |
| A40 | 0.00% | 88.26% | 0.00% | 7.03% | pregnant | pregnant | IVF |
| B53 | 0.00% | 74.76% | 0.00% | 7.77% | pregnant | pregnant | ICSI |
| F26 | 0.00% | 61.78% | 0.00% | 8.36% | pregnant | pregnant | IVF |
| A23 | 0.00% | 84.41% | 32.28% | 14.15% | pregnant | pregnant | ICSI |
| A26 | 0.00% | 66.14% | 0.00% | 19.43% | pregnant | pregnant | ICSI |
| F35 | 0.00% | 95.34% | 0.00% | 4.14% | pregnant | not pregnant | ICSI |
| B36 | 0.00% | 92.76% | 2.70% | 0.00% | pregnant | not pregnant | ICSI |

(continued)

| Sample ID | G. vaginalis | Total Lactobacillus | L. jensenii | Total Proteobacterium | Prediction | Outcome | Treatment |
|---|---|---|---|---|---|---|---|
| B85 | 0.00% | 71.82% | 0.00% | 0.62% | pregnant | pregnant | ICSI |
| A10 | 0.00% | 87.21% | 8.77% | 12.79% | pregnant | not pregnant | IVF |
| B19 | 0.00% | 98.50% | 10.85% | 1.50% | pregnant | not pregnant | ICSI |
| C42 | 0.00% | 92.65% | 0.00% | 7.35% | pregnant | not pregnant | ICSI |
| E18 | 0.00% | 98.99% | 0.00% | 1.01% | pregnant | not pregnant | IVF |
| A47 | 0.00% | 91.06% | 1.65% | 8.94% | pregnant | not pregnant | ICSI |
| B70 | 0.00% | 96.30% | 6.57% | 3.70% | pregnant | not pregnant | ICSI |
| E36 | 0.00% | 84.73% | 8.64% | 5.38% | pregnant | not pregnant | ICSI |
| F20 | 0.00% | 77.54% | 0.00% | 22.46% | pregnant | pregnant | ICSI |
| F1 | 0.00% | 85.95% | 33.17% | 14.05% | pregnant | pregnant | ICSI |
| H8 | 0.00% | 93.77% | 3.65% | 4.53% | pregnant | not pregnant | IVF |
| C55 | 0.00% | 21.54% | 0.00% | 16.63% | pregnant | pregnant | ICSI |
| F8 | 0.00% | 75.26% | 0.00% | 18.63% | pregnant | pregnant | ICSI |
| B47 | 0.00% | 79.87% | 9.26% | 18.41% | pregnant | not pregnant | ICSI |
| C28 | 0.00% | 96.89% | 9.70% | 2.06% | pregnant | pregnant | ICSI |
| B7 | 0.00% | 98.16% | 10.54% | 0.77% | pregnant | not pregnant | ICSI |
| B55 | 0.00% | 99.64% | 31.26% | 0.00% | pregnant | pregnant | ICSI |
| C18 | 0.00% | 98.72% | 1.22% | 0.00% | pregnant | not pregnant | ICSI |
| E32 | 0.00% | 93.00% | 7.61% | 0.00% | pregnant | pregnant | IVF |
| A8 | 0.00% | 90.23% | 0.00% | 9.41% | pregnant | not pregnant | IVF |
| F5 | 0.00% | 78.48% | 4.45% | 19.45% | pregnant | pregnant | IVF |
| F11 | 0.00% | 67.61% | 0.00% | 27.32% | pregnant | pregnant | ICSI |
| H6 | 0.00% | 97.93% | 6.39% | 0.93% | pregnant | pregnant | IVF |
| B39 | 0.00% | 88.13% | 0.00% | 11.09% | pregnant | pregnant | IVF |
| E59 | 0.00% | 81.37% | 4.64% | 9.61% | pregnant | pregnant | IVF |

| Sample ID | G. vaginalis | Total Lactobacillus | L. jensenii | Total Proteobacterium | Prediction | Outcome | Treatment |
|---|---|---|---|---|---|---|---|
| G1 | 0.00% | 30.30% | 0.00% | 5.33% | pregnant | not pregnant | ICSI |
| E29 | 0.00% | 95.59% | 7.00% | 0.00% | pregnant | not pregnant | ICSI |
| A29 | 0.00% | 43.74% | 0.00% | 16.69% | pregnant | pregnant | IVF |
| B27 | 0.00% | 99.52% | 0.00% | 0.00% | pregnant | not pregnant | IVF |
| B25 | 0.00% | 99.41% | 1.22% | 0.00% | pregnant | not pregnant | ICSI |
| C51 | 0.00% | 53.99% | 3.71% | 13.15% | pregnant | pregnant | ICSI |
| E48 | 0.00% | 93.85% | 0.00% | 6.15% | pregnant | pregnant | ICSI |
| C60 | 0.00% | 66.96% | 0.00% | 0.56% | pregnant | pregnant | ICSI |
| D9 | 0.00% | 99.14% | 7.00% | 0.00% | pregnant | not pregnant | ICSI |
| E39 | 0.00% | 99.52% | 12.88% | 0.00% | pregnant | not pregnant | IVF |
| A42 | 0.00% | 47.33% | 0.48% | 27.49% | pregnant | not pregnant | IVF |
| B4 | 0.00% | 94.75% | 0.00% | 2.23% | pregnant | not pregnant | IVF |
| B10 | 0.00% | 74.81% | 5.17% | 3.21% | pregnant | not pregnant | ICSI |
| E49 | 0.00% | 93.07% | 13.74% | 6.93% | pregnant | not pregnant | ICSI |
| B61 | 0.00% | 43.79% | 0.00% | 16.35% | pregnant | not pregnant | ICSI |
| B20 | 0.00% | 98.72% | 0.00% | 0.81% | pregnant | not pregnant | ICSI |
| E19 | 0.00% | 97.20% | 0.00% | 0.00% | pregnant | pregnant | ICSI |
| F2 | 0.00% | 53.49% | 0.00% | 17.92% | pregnant | not pregnant | IVF |
| A45 | 0.00% | 84.93% | 0.00% | 14.02% | pregnant | not pregnant | IVF |
| F41 | 0.00% | 46.28% | 0.00% | 0.00% | pregnant | pregnant | ICSI |
| F31 | 0.00% | 92.91% | 11.40% | 6.48% | pregnant | not pregnant | IVF |
| F21 | 0.00% | 94.22% | 0.00% | 5.78% | pregnant | pregnant | ICSI |
| A4 | 0.00% | 86.95% | 5.54% | 8.72% | pregnant | not pregnant | IVF |
| E50 | 0.00% | 95.47% | 2.79% | 4.53% | pregnant | pregnant | ICSI |
| F39 | 0.00% | 96.02% | 0.55% | 3.55% | pregnant | pregnant | ICSI |

EP 3 797 174 B1

| Sample ID | G. vaginalis | Total Lactobacillus | L. jensenii | Total Proteobacterium | Prediction | Outcome | Treatment |
|---|---|---|---|---|---|---|---|
| C13 | 0.00% | 91.98% | 2.47% | 0.00% | pregnant | pregnant | ICSI |
| B14 | 0.00% | 97.03% | 1.84% | 1.31% | pregnant | not pregnant | ICSI |
| C21 | 0.00% | 100.00% | 0.00% | 0.00% | pregnant | not pregnant | IVF |
| F24 | 0.00% | 94.81% | 1.60% | 4.52% | pregnant | not pregnant | ICSI |
| C15 | 0.00% | 77.16% | 18.79% | 0.00% | pregnant | not pregnant | ICSI |
| D22 | 0.00% | 89.97% | 0.00% | 10.03% | pregnant | pregnant | IVF |
| C56 | 0.00% | 100.00% | 34.59% | 0.00% | pregnant | pregnant | ICSI |
| C38 | 0.00% | 86.50% | 20.08% | 11.42% | pregnant | pregnant | ICSI |
| B69 | 0.00% | 95.88% | 28.43% | 0.00% | pregnant | pregnant | ICSI |
| B78 | 0.00% | 92.96% | 0.00% | 4.72% | pregnant | pregnant | ICSI |
| A24 | 0.00% | 84.77% | 13.68% | 13.93% | pregnant | not pregnant | IVF |
| C40 | 0.00% | 87.50% | 0.00% | 12.50% | pregnant | not pregnant | IVF |
| C43 | 0.00% | 88.19% | 14.75% | 11.41% | pregnant | not pregnant | ICSI |
| A2 | 0.00% | 80.15% | 7.53% | 19.07% | pregnant | pregnant | ICSI |
| E52 | 0.00% | 88.93% | 0.00% | 7.08% | pregnant | pregnant | ICSI |
| C31 | 0.00% | 98.89% | 24.03% | 1.11% | pregnant | pregnant | ICSI |
| A25 | 0.00% | 97.50% | 22.55% | 1.39% | pregnant | pregnant | IVF |
| C49 | 0.00% | 75.18% | 0.00% | 8.93% | pregnant | pregnant | ICSI |
| B87 | 0.00% | 75.19% | 0.00% | 20.94% | pregnant | not pregnant | ICSI |
| D14 | 0.00% | 93.16% | 0.00% | 5.35% | pregnant | not pregnant | IVF |
| B9 | 0.00% | 96.41% | 0.00% | 3.00% | pregnant | pregnant | ICSI |
| C30 | 0.00% | 96.07% | 0.00% | 1.94% | pregnant | pregnant | ICSI |
| A13 | 0.00% | 85.00% | 4.65% | 12.89% | pregnant | pregnant | IVF |
| F22 | 0.00% | 80.52% | 0.00% | 7.63% | pregnant | not pregnant | ICSI |
| D20 | 0.00% | 94.40% | 0.00% | 5.60% | pregnant | not pregnant | IVF |

(continued)

| Sample ID | G. vaginalis | Total Lactobacillus | L. jensenii | Total Proteobacterium | Prediction | Outcome | Treatment |
|---|---|---|---|---|---|---|---|
| A30 | 0.00% | 82.46% | 0.00% | 15.75% | pregnant | not pregnant | ICSI |
| A43 | 0.00% | 81.82% | 0.00% | 16.76% | pregnant | pregnant | ICSI |
| F30 | 0.00% | 83.95% | 0.00% | 15.28% | pregnant | not pregnant | IVF/ICSI |
| A14 | 0.00% | 84.41% | 0.00% | 10.73% | pregnant | not pregnant | ICSI |
| B38 | 0.00% | 88.61% | 0.00% | 10.61% | pregnant | pregnant | IVF |
| A12 | 0.00% | 90.33% | 0.00% | 8.55% | pregnant | not pregnant | IVF |
| D15 | 0.00% | 89.45% | 0.00% | 9.06% | pregnant | not pregnant | ICSI |
| A7 | 0.00% | 89.86% | 0.00% | 8.26% | pregnant | pregnant | ICSI |
| F14 | 0.00% | 99.13% | 0.00% | 0.00% | pregnant | not pregnant | ICSI |
| F19 | 0.00% | 90.53% | 0.00% | 8.87% | pregnant | not pregnant | ICSI |
| B33 | 0.00% | 90.60% | 0.00% | 8.81% | pregnant | not pregnant | IVF |
| C23 | 0.00% | 97.67% | 7.02% | 1.79% | pregnant | not pregnant | ICSI |
| B77 | 0.00% | 96.25% | 5.11% | 0.00% | pregnant | pregnant | ICSI |
| B42 | 0.00% | 93.63% | 0.00% | 2.01% | pregnant | pregnant | ICSI |
| B57 | 0.00% | 97.90% | 0.00% | 0.00% | pregnant | pregnant | IVF |
| B24 | 0.00% | 96.73% | 0.00% | 0.00% | pregnant | not pregnant | ICSI |
| B5 | 0.00% | 96.86% | 0.00% | 1.46% | pregnant | not pregnant | ICSI |

Table 4: Microbial composition of vaginal flora; prediction of chance of failure of ART procedure.

| Sample ID | L.crispatus | L. iners | Prediction | Outcome | Treatment |
|---|---|---|---|---|---|
| F20 | 58.91% | 18.63% | pregnant | pregnant | ICSI |
| B39 | 58.24% | 29.89% | pregnant | pregnant | IVF |
| E48 | 57.83% | 36.02% | pregnant | pregnant | ICSI |
| E32 | 57.50% | 27.89% | pregnant | pregnant | IVF |
| B85 | 57.46% | 2.51% | pregnant | pregnant | ICSI |
| E19 | 52.87% | 44.33% | pregnant | pregnant | ICSI |
| F8 | 52.73% | 22.53% | pregnant | pregnant | ICSI |
| A23 | 52.13% | 0.00% | pregnant | pregnant | ICSI |
| C14 | 49.36% | 0.00% | pregnant | pregnant | ICSI |
| E59 | 46.47% | 30.27% | pregnant | pregnant | IVF |
| F5 | 44.90% | 29.12% | pregnant | pregnant | IVF |
| F39 | 42.43% | 53.04% | pregnant | pregnant | ICSI |
| B55 | 42.17% | 26.20% | pregnant | pregnant | ICSI |
| B72 | 40.99% | 0.00% | pregnant | pregnant | ICSI |
| E50 | 40.00% | 52.68% | pregnant | pregnant | ICSI |
| F11 | 38.29% | 29.32% | pregnant | pregnant | ICSI |
| C13 | 36.40% | 53.11% | pregnant | pregnant | ICSI |
| F1 | 33.57% | 19.22% | pregnant | pregnant | ICSI |
| A26 | 30.90% | 0.00% | pregnant | pregnant | ICSI |
| C60 | 30.73% | 36.24% | pregnant | pregnant | ICSI |
| F26 | 23.83% | 0.00% | pregnant | pregnant | IVF |
| A5 | 20.93% | 0.00% | pregnant | pregnant | ICSI |
| B9 | 16.84% | 79.57% | pregnant | pregnant | ICSI |
| C30 | 16.34% | 79.73% | pregnant | pregnant | ICSI |
| E52 | 16.23% | 72.70% | pregnant | pregnant | ICSI |
| C51 | 14.89% | 35.39% | pregnant | pregnant | ICSI |
| B40 | 4.24% | 0.00% | pregnant | pregnant | ICSI |
| B57 | 3.34% | 94.56% | pregnant | pregnant | IVF |
| B30 | 2.77% | 0.00% | pregnant | pregnant | ICSI |
| B84 | 0.55% | 0.00% | pregnant | pregnant | ICSI |
| F36 | 0.00% | 0.00% | pregnant | pregnant | ICSI |
| B76 | 73.00% | 27.00% | pregnant | pregnant | IVF |
| A40 | 0.00% | 0.00% | pregnant | pregnant | IVF |
| C55 | 0.00% | 21.54% | pregnant | pregnant | ICSI |
| A29 | 0.00% | 31.04% | pregnant | pregnant | IVF |
| F41 | 0.00% | 46.28% | pregnant | pregnant | ICSI |
| F21 | 0.00% | 50.96% | pregnant | pregnant | ICSI |

(continued)

| Sample ID | L.crispatus | L. iners | Prediction | Outcome | Treatment |
|---|---|---|---|---|---|
| D22 | 0.00% | 65.08% | pregnant | pregnant | IVF |
| C56 | 0.00% | 65.41% | pregnant | pregnant | ICSI |
| C38 | 0.00% | 66.42% | pregnant | pregnant | ICSI |
| B69 | 0.00% | 67.45% | pregnant | pregnant | ICSI |
| B78 | 0.00% | 70.19% | pregnant | pregnant | ICSI |
| A2 | 0.00% | 72.62% | pregnant | pregnant | ICSI |
| C31 | 0.00% | 74.86% | pregnant | pregnant | ICSI |
| A25 | 0.00% | 74.95% | pregnant | pregnant | IVF |
| C49 | 0.00% | 75.18% | pregnant | pregnant | ICSI |
| A13 | 0.00% | 80.34% | pregnant | pregnant | IVF |
| A43 | 0.00% | 81.82% | pregnant | pregnant | ICSI |
| B38 | 0.00% | 88.61% | pregnant | pregnant | IVF |
| A7 | 0.00% | 89.86% | pregnant | pregnant | ICSI |
| B77 | 0.00% | 91.14% | pregnant | pregnant | ICSI |
| B42 | 0.00% | 93.63% | pregnant | pregnant | ICSI |
| E29 | 58.01% | 30.58% | pregnant | not pregnant | ICSI |
| E36 | 57.47% | 18.62% | pregnant | not pregnant | ICSI |
| B4 | 55.67% | 39.08% | pregnant | not pregnant | IVF |
| D9 | 55.28% | 36.86% | pregnant | not pregnant | ICSI |
| B20 | 54.81% | 43.91% | pregnant | not pregnant | ICSI |
| C20 | 53.99% | 0.00% | pregnant | not pregnant | ICSI |
| B27 | 51.55% | 31.46% | pregnant | not pregnant | IVF |
| E39 | 49.12% | 37.51% | pregnant | not pregnant | IVF |
| B47 | 47.04% | 23.57% | pregnant | not pregnant | ICSI |
| C21 | 42.67% | 57.33% | pregnant | not pregnant | IVF |
| B14 | 40.89% | 54.30% | pregnant | not pregnant | ICSI |
| A45 | 39.73% | 45.20% | pregnant | not pregnant | IVF |
| E49 | 36.14% | 43.19% | pregnant | not pregnant | ICSI |
| F24 | 35.67% | 57.54% | pregnant | not pregnant | ICSI |
| F31 | 32.89% | 48.63% | pregnant | not pregnant | IVF |
| A4 | 30.38% | 51.03% | pregnant | not pregnant | IVF |
| B10 | 29.68% | 39.96% | pregnant | not pregnant | ICSI |
| D14 | 16.50% | 76.65% | pregnant | not pregnant | IVF |
| C40 | 15.67% | 71.83% | pregnant | not pregnant | IVF |
| D20 | 13.13% | 81.27% | pregnant | not pregnant | IVF |
| A42 | 9.19% | 37.67% | pregnant | not pregnant | IVF |
| F2 | 9.03% | 44.46% | pregnant | not pregnant | IVF |
| A20 | 2.27% | 0.00% | pregnant | not pregnant | ICSI |

(continued)

| Sample ID | L.crispatus | L. iners | Prediction | Outcome | Treatment |
|---|---|---|---|---|---|
| C43 | 1.53% | 71.91% | pregnant | not pregnant | ICSI |
| A12 | 1.39% | 88.93% | pregnant | not pregnant | IVF |
| A30 | 1.11% | 81.34% | pregnant | not pregnant | ICSI |
| C4 | 0.00% | 0.00% | pregnant | not pregnant | IVF |
| G3 | 82.00% | 0.00% | pregnant | not pregnant | IVF |
| F33 | 45.00% | 0.00% | pregnant | not pregnant | ICSI |
| F42 | 73.00% | 4.00% | pregnant | not pregnant | IVF |
| E7 | 62.00% | 10.00% | pregnant | not pregnant | IVF |
| E8 | 93.62% | 0.00% | pregnant | not pregnant | IVF |
| A22 | 0.00% | 0.00% | pregnant | not pregnant | ICSI |
| F9 | 0.00% | 0.00% | pregnant | not pregnant | IVF |
| G1 | 0.00% | 30.30% | pregnant | not pregnant | ICSI |
| B61 | 0.00% | 43.79% | pregnant | not pregnant | ICSI |
| C15 | 0.00% | 58.38% | pregnant | not pregnant | ICSI |
| A24 | 0.00% | 71.08% | pregnant | not pregnant | IVF |
| B87 | 0.00% | 75.19% | pregnant | not pregnant | ICSI |
| F22 | 0.00% | 80.52% | pregnant | not pregnant | ICSI |
| F30 | 0.00% | 83.95% | pregnant | not pregnant | IVF/ICSI |
| A14 | 0.00% | 84.41% | pregnant | not pregnant | ICSI |
| D15 | 0.00% | 89.45% | pregnant | not pregnant | ICSI |
| F14 | 0.00% | 90.12% | pregnant | not pregnant | ICSI |
| F19 | 0.00% | 90.53% | pregnant | not pregnant | ICSI |
| B33 | 0.00% | 90.60% | pregnant | not pregnant | IVF |
| C23 | 0.00% | 90.65% | pregnant | not pregnant | ICSI |
| B24 | 0.00% | 96.73% | pregnant | not pregnant | ICSI |
| B5 | 0.00% | 96.86% | pregnant | not pregnant | ICSI |
| D3 | 10.82% | 23.76% | pregnant | pregnant | ICSI |
| H7 | 4.06% | 13.42% | pregnant | pregnant | IVF |
| H3 | 55.17% | 0.00% | pregnant | not pregnant | IVF |
| B17 | 36.44% | 0.00% | pregnant | not pregnant | ICSI |
| F32 | 19.15% | 57.74% | pregnant | not pregnant | ICSI |
| B59 | 15.42% | 0.00% | pregnant | not pregnant | ICSI |
| A41 | 9.26% | 43.53% | pregnant | not pregnant | ICSI |
| B65 | 4.55% | 19.04% | pregnant | not pregnant | ICSI |
| B45 | 2.78% | 34.85% | pregnant | not pregnant | IVF |
| B41 | 1.41% | 67.16% | pregnant | not pregnant | IVF |
| A32 | 1.40% | 14.54% | pregnant | not pregnant | ICSI |
| F34 | 0.00% | 0.00% | pregnant | not pregnant | IVF |

(continued)

| Sample ID | L.crispatus | L. iners | Prediction | Outcome | Treatment |
|---|---|---|---|---|---|
| B83 | 0.00% | 0.00% | pregnant | not pregnant | ICSI |
| C1 | 0.00% | 0.00% | pregnant | not pregnant | ICSI |
| H9 | 95.51% | 0.00% | pregnant | not pregnant | IVF |
| B13 | 0.00% | 0.00% | pregnant | not pregnant | ICSI |
| B15 | 0.00% | 0.00% | pregnant | not pregnant | IVF |
| F40 | 0.00% | 0.00% | pregnant | not pregnant | IVF |
| A18 | 0.00% | 0.00% | pregnant | not pregnant | IVF |
| C25 | 0.00% | 0.00% | pregnant | not pregnant | ICSI |
| B11 | 0.00% | 0.00% | pregnant | not pregnant | ICSI |
| F23 | 0.00% | 0.00% | pregnant | not pregnant | ICSI |
| B88 | 0.00% | 0.00% | pregnant | not pregnant | ICSI |
| F6 | 0.00% | 0.00% | pregnant | not pregnant | IVF |
| B18 | 0.00% | 14.88% | pregnant | not pregnant | ICSI |
| C11 | 0.00% | 24.40% | pregnant | not pregnant | ICSI |
| B54 | 0.00% | 31.39% | pregnant | not pregnant | ICSI |
| D2 | 0.00% | 43.93% | pregnant | not pregnant | ICSI |
| C47 | 0.00% | 45.01% | pregnant | not pregnant | ICSI |
| C10 | 0.00% | 49.67% | pregnant | not pregnant | ICSI |
| F28 | 0.00% | 53.18% | pregnant | not pregnant | ICSI |
| F10 | 0.00% | 59.16% | pregnant | not pregnant | IVF |
| E38 | 0.00% | 59.48% | pregnant | not pregnant | ICSI |
| B64 | 99.21% | 0.00% | Not pregnant | pregnant | ICSI |
| C33 | 97.29% | 0.00% | Not pregnant | pregnant | IVF |
| B12 | 96.88% | 0.00% | Not pregnant | pregnant | IVF |
| B6 | 96.10% | 0.00% | Not pregnant | pregnant | ICSI |
| B44 | 91.38% | 0.00% | Not pregnant | pregnant | ICSI |
| C36 | 91.17% | 0.00% | Not pregnant | pregnant | ICSI |
| F37 | 85.77% | 0.00% | Not pregnant | pregnant | ICSI |
| B37 | 77.53% | 0.00% | Not pregnant | pregnant | ICSI |
| B53 | 74.76% | 0.00% | Not pregnant | pregnant | ICSI |
| B3 | 70.86% | 0.00% | Not pregnant | pregnant | IVF |
| A3 | 69.20% | 0.00% | Not pregnant | pregnant | ICSI |
| C28 | 63.01% | 24.18% | Not pregnant | pregnant | ICSI |
| H6 | 61.85% | 29.68% | Not pregnant | pregnant | IVF |
| B26 | 100.00% | 0.00% | Not pregnant | not pregnant | ICSI |
| D6 | 100.00% | 0.00% | Not pregnant | not pregnant | IVF |
| B28 | 98.21% | 0.00% | Not pregnant | not pregnant | ICSI |
| B29 | 98.08% | 0.00% | Not pregnant | not pregnant | ICSI |

(continued)

| Sample ID | L.crispatus | L. iners | Prediction | Outcome | Treatment |
|-----------|-------------|----------|------------|---------|-----------|
| E47 | 97.84% | 0.00% | Not pregnant | not pregnant | IVF |
| B63 | 95.09% | 0.00% | Not pregnant | not pregnant | ICSI |
| B49 | 94.75% | 0.00% | Not pregnant | not pregnant | ICSI |
| F35 | 93.59% | 1.74% | Not pregnant | not pregnant | ICSI |
| B67 | 93.28% | 0.00% | Not pregnant | not pregnant | ICSI |
| H5 | 92.35% | 0.00% | Not pregnant | not pregnant | ICSI |
| F15 | 92.32% | 0.00% | Not pregnant | not pregnant | IVF |
| E57 | 91.98% | 0.00% | Not pregnant | not pregnant | ICSI |
| B71 | 91.45% | 0.00% | Not pregnant | not pregnant | IVF |
| A15 | 91.16% | 0.00% | Not pregnant | not pregnant | IVF |
| F13 | 90.41% | 0.00% | Not pregnant | not pregnant | IVF |
| F12 | 89.56% | 0.00% | Not pregnant | not pregnant | ICSI |
| A19 | 88.73% | 0.00% | Not pregnant | not pregnant | ICSI |
| B36 | 88.02% | 2.04% | Not pregnant | not pregnant | ICSI |
| E44 | 87.27% | 0.00% | Not pregnant | not pregnant | IVF |
| F17 | 83.96% | 0.00% | Not pregnant | not pregnant | IVF |
| B19 | 83.50% | 4.15% | Not pregnant | not pregnant | ICSI |
| E18 | 81.59% | 17.40% | Not pregnant | not pregnant | IVF |
| F38 | 80.94% | 0.00% | Not pregnant | not pregnant | ICSI |
| E40 | 80.71% | 0.00% | Not pregnant | not pregnant | ICSI |
| B51 | 78.65% | 0.00% | Not pregnant | not pregnant | ICSI |
| A16 | 77.71% | 0.00% | Not pregnant | not pregnant | ICSI |
| C32 | 77.60% | 0.00% | Not pregnant | not pregnant | IVF |
| C42 | 77.31% | 15.35% | Not pregnant | not pregnant | ICSI |
| B90 | 76.46% | 0.00% | Not pregnant | not pregnant | ICSI |
| D23 | 75.21% | 0.00% | Not pregnant | not pregnant | ICSI |
| C3 | 74.35% | 0.00% | Not pregnant | not pregnant | IVF |
| C39 | 72.78% | 0.00% | Not pregnant | not pregnant | ICSI |
| B70 | 71.92% | 17.81% | Not pregnant | not pregnant | ICSI |
| A47 | 71.75% | 17.65% | Not pregnant | not pregnant | ICSI |
| H8 | 70.75% | 19.37% | Not pregnant | not pregnant | IVF |
| C18 | 70.00% | 27.50% | Not pregnant | not pregnant | ICSI |
| D21 | 69.64% | 0.00% | Not pregnant | not pregnant | ICSI |
| A21 | 65.89% | 0.00% | Not pregnant | not pregnant | ICSI |
| A36 | 65.38% | 0.00% | Not pregnant | not pregnant | ICSI |
| B25 | 65.07% | 33.12% | Not pregnant | not pregnant | ICSI |
| A10 | 63.73% | 2.71% | Not pregnant | not pregnant | IVF |
| B7 | 62.95% | 24.66% | Not pregnant | not pregnant | ICSI |

(continued)

| Sample ID | L.crispatus | L. iners | Prediction | Outcome | Treatment |
|-----------|-------------|----------|------------|---------|-----------|
| A8 | 62.17% | 28.05% | Not pregnant | not pregnant | IVF |
| C53 | 61.89% | 0.00% | Not pregnant | not pregnant | ICSI |
| F16 | 62.27% | 0.00% | Not pregnant | not pregnant | ICSI |

**Claims**

1. A method for predicting the likelihood that an assisted reproductive technology (ART) procedure will not result in a pregnancy, wherein a sample from a female mammalian subject taken before or during the ART procedure, is analyzed for:

   a) the presence of Gardnerella vaginalis IST1,
   b) relative abundance of Lactobacillus species,
   c) relative abundance of Lactobacillus jensenii, and
   d) relative abundance of Proteobacteria,
   and wherein it is concluded that the subject has a high likelihood of not becoming pregnant as a result of the ART procedure if at least one of the following applies:

   I. the sample comprises Gardnerella vaginalis IST1;
   II. the relative abundance of Lactobacillus species is below a value of 20%;
   III. the relative abundance of Lactobacillus jensenii is above a value of 35%; or
   IV. the relative abundance of Proteobacteria is above a value of 28%,

   wherein Gardnerella vaginalis IST1 is defined as a species of Gardnerella vaginalis that results in a specific IS-fragment with a length of 428, 429 or 430 nucleotides when amplified with primers according to SEQ ID NO: 1 and SEQ ID NO: 2, to be applied to a vaginal microbial analysis, wherein the sample is a vaginal swab or a urine sample.

2. The method according to claim 1 wherein the presence of Gardnerella vaginalis IST1 is determined by a quantitative polymerase chain reaction (PCR).

3. The method according claim 1 or 2 wherein the mammalian subject is human.

4. The method according to any one of claims 1 - 3 wherein the ART procedure is an in vitro fertilization (IVF) procedure, such as intra-cytoplasmic sperm injection (ICSI) procedure.

**Patentansprüche**

1. Verfahren zur Vorhersage der Wahrscheinlichkeit, dass eine ART(Assisted Reproductive Technology)-Maßnahme zu keiner Schwangerschaft führt, wobei eine einem weiblichen Säugerindividuum vor oder während der ART-Maßnahme entnommene Probe auf Folgendes analysiert wird:

   a) das Vorliegen von Gardnerella vaginalis IST1,
   b) relative Häufigkeit von Lactobacillus-Spezies,
   c) relative Häufigkeit von Lactobacillus jensenii und
   d) relative Häufigkeit von Proteobacteria,

   und wobei der Schluss gezogen wird, dass bei dem Individuum eine hohe Wahrscheinlichkeit besteht, dass die ART-Maßnahme zu keiner Schwangerschaft führt, falls wenigstens einer der folgenden Punkte zutrifft:

   I. die Probe enthält Gardnerella vaginalis IST 1;
   II. die relative Häufigkeit von Lactobacillus-Spezies liegt unterhalb eines Werts von 20 %;

III. die relative Häufigkeit von Lactobacillus jensenii liegt oberhalb eines Werts von 35 %; oder

IV. die relative Häufigkeit von Proteobacteria liegt oberhalb eines Werts von 28 %, wobei Gardnerella vaginalis IST1 als eine Spezies von Gardnerella vaginalis definiert ist, die ein spezifisches IS-Fragment mit einer Länge von 428, 429 oder 430 Nukleotiden bei Amplifikation mit Primern gemäß SEQ ID NO: 1 und SEQ ID NO: 2, die auf eine Vaginom-Analyse angewandt werden, ergibt, wobei es sich bei der Probe um einen Vaginalabstrich oder eine Urinprobe handelt.

2. Verfahren nach Anspruch 1, wobei das Vorliegen von Gardnerella vaginalis IST1 mit einer quantitativen PCR (Polymerase Chain Reaction) bestimmt wird.

3. Verfahren nach Anspruch 1 oder 2, wobei es sich bei dem Säugerindividuum um einen Menschen handelt.

4. Verfahren nach einem der Ansprüche 1 - 3, wobei es sich bei der ART-Maßnahme um eine IVF(In Vitro Fertilization)-Maßnahme wie ICSI(Intra-Cytoplasmic Sperm Injection)-Maßnahme handelt.

**Revendications**

1. Procédé de prédiction de la probabilité qu'une procédure de procréation médicalement assistée (ART) n'aboutisse pas à une grossesse, dans lequel un échantillon prélevé sur un sujet mammifère femelle avant ou pendant la procédure ART est analysé pour y rechercher :

a) la présence de Gardnerella vaginalis IST1,
b) l'abondance relative d'espèces de Lactobacillus,
c) l'abondance relative de Lactobacillus jensenii, et
d) l'abondance relative de protéobactéries,

et dans lequel il est conclu que le sujet présente une forte probabilité de ne pas tomber enceinte à la suite de la procédure ART si au moins l'un de ce qui suit s'applique :

I. l'échantillon comprend Gardnerella vaginalis IST1 ;
II. l'abondance relative des espèces de Lactobacillus est inférieure à une valeur de 20% ;
III. l'abondance relative de Lactobacillus jensenii est supérieure à une valeur de 35% ; ou
IV. l'abondance relative des protéobactéries est supérieure à une valeur de 28%, dans laquelle Gardnerella vaginalis IST1 est définie comme une espèce de Gardnerella vaginalis qui produit un fragment IS spécifique d'une longueur de 428, 429 ou 430 nucléotides lorsqu'il est amplifié avec des amorces conformes à SEQ ID NO: 1 et SEQ ID NO: 2, à appliquer à une analyse microbienne vaginale, dans lequel l'échantillon est un écouvillon vaginal ou un échantillon d'urine.

2. Procédé selon la revendication 1, dans lequel la présence de Gardnerella vaginalis IST1 est déterminée par une réaction en chaîne par polymérase quantitative (PCR).

3. Procédé selon la revendication 1 ou 2, dans lequel le sujet mammifère est humain.

4. Procédé selon l'une quelconque des revendications 1 à 3, dans lequel la procédure ART est une procédure de fécondation in vitro (FIV), telle qu'une procédure d'injection intracytoplasmique de spermatozoïdes (ICSI).

Figure 1

**EP 3 797 174 B1**

## REFERENCES CITED IN THE DESCRIPTION

**Patent documents cited in the description**

- EP 2742359 B1
- WO 2013025095 A1
- EP 1985712 A1

**Non-patent literature cited in the description**

- **EVERS, J.L.** *Lancet,* 2002, vol. 360, 151-159
- **ANDERSEN, A. et al.** *Hum. Reprod.,* 2007, vol. 22, 1513-1525
- **TEMPLETON, W. et al.** *Lancet,* 1996, vol. 348, 1402-1406
- **NELSON, S.M. ; LAWLOR D.A.** *PLOS Medicine,* 2011, vol. 8, 1-10
- **SELMAN et al.** *J. Assisted Reproduction and Genetics,* 2007, vol. 24, 395-399
- **HAAHR et al.** *Human Reproduction,* 2016, vol. 31, 795-803
- **MANGOT-BERTRAND et al.** *Eur.J. Clin. Microbiol. and Infectious Diseases,* 2012, vol. 32, 535-54
- **SELMAN et al.** *J. assisted Reproduction and Genetics,* 2007, vol. 24, 395-399
- **ECKERT et al.** *Inf. Disease in obstetrics and Gynecology,* 2003, vol. 11, 11-17
- **BUDDING, E. et al.** *J. Clin. Microbiol.,* 2016, vol. 54, 934-943
- **BUDDING AE ; HOOGEWERF M ; VANDEN-BROUCKE-GRAULS CM ; SAVELKOUL PH.** *J Clin Microbiol.,* 13 January 2016, vol. 54 (4), 934-43